# EUROPÄISCHE PATENTSCHRIFT

(11) **EP 1 227 851 B1**
(45) Veröffentlichungstag und Bekanntmachung des Hinweises auf die Patenterteilung: **07.05.2003**
(21) Anmeldenummer: 00975867.3
(22) Anmeldetag: 14.10.2000
(51) Int. Cl.: A61L 24/00, A61L 27/58, A61L 27/42

(54) **RESORBIERBARES KNOCHEN-IMPLANTATMATERIAL SOWIE VERFAHREN ZUR HERSTELLUNG DESSELBEN**
ABSORBABLE BONE IMPLANT MATERIAL AND METHOD FOR PRODUCING THE SAME
MATERIAU D'IMPLANT OSSEUX RESORBABLE ET SON PROCEDE DE PRODUCTION

(30) Priorität: 09.11.1999 DE 19953771
(43) Veröffentlichungstag der Anmeldung: 07.08.2002
(73) Patentinhaber: Coripharm Medizinprodukte GmbH & Co. KG., 64807 Dieburg (DE)
(72) Erfinder: WAHLIG, Helmut, 64287 Darmstadt (DE); DINGELDEIN, Elvira, 63303 Dreieich (DE); WÜST, Edgar, 63110 Rodgau (DE); SATTIG, Christoph, 64807 Dieburg (DE)
(74) Vertreter: Helber, Friedrich G., Dipl.-Ing.
(86) Internationale Anmeldenummer: EP0010133
(87) Internationale Veröffentlichungsnummer: WO01034216

(56) Entgegenhaltungen:
- EP-A- 0 664 133
- WO-A-87/05521
- US-A- 4 619 655
- US-A- 5 824 087

## Beschreibung

Die Erfindung betrifft ein aus einer Hydroxylapatit enthaltenden pulverförmigen Komponente und Flüssigkeit aufbereitetes resorbierbares Knochen-Implantatmaterial sowie ein Verfahren zur Herstellung desselben.

Die Bedeutung der Knochenersatzmaterialien, insbesondere in den Bereichen Orthopädie, Traumatologie, Schädel-, Kiefer- und Gesichtschirurgie, sowie Ododontologie nimmt noch immer zu. Wesentliche Einsatzgebiete - sowohl in der Human- als auch in der Veterinärmedizin - sind beispielsweise die Überbrückung großer Knochendefekte bei Trümmerfrakturen sowie die Fixation kleiner Knochensplitter, das Auffüllen von Knochendefekten bei Knochenzysten und nach der Beseitigung von Knochentumoren, das Auffüllen von Hohlräumen bei chronischer Osteomyelitis, der Einsatz bei Substanzverlusten an Alveolen oder Kieferknochen und die Verwendung als Trägermaterial, z.B. für Antibiotika, Zytostatika und osteogene Substanzen. Ihrem breiten Indikationsgebiet entspricht die weltweit von Jahr zu Jahr steigende Zahl der Anwendungen von Knochenersatzmaterialien. Einige Gründe hierfür sind die steigende Lebenserwartung, die zunehmende Industrialisierung, die steigende Verkehrsdichte und immer bessere chirurgische Techniken, die einen immer breiteren Einsatz dieser Materialien möglich machen. Der Bedarf an Knochenersatzmaterialien ist deshalb so groß, weil das in dieser Hinsicht am besten geeignete Material, die autogene Spongiosa, nur in sehr begrenztem Maße zur Verfügung steht. Außerdem bedingt deren Anwendung stets eine zweite, zusätzliche Operation, die ihrerseits mit allen medizinisch-chirurgischen Risiken eines operativen Eingriffes verbunden ist, wobei der dabei entstehende Defekt in den meisten Fällen wiederum einer Auffüllung - mit einem Knochenersatzmaterial - bedarf.

Allogener Knochen - als nächstbestes Implantatmaterial - ist heute kaum noch einsetzbar, weil dabei ein hohes Risiko für eine Übertragung infektiösen Materials wie u.a. Hepatitis- und HIV-Viren, oder die Kreutzfeld-Jacob-Erkrankung besteht und die Bereitstellung eines entsprechend geprüften, unbedenklichen Materials, sowie die damit verbundene Betreibung von Knochenbanken, einen kaum zu erbringenden finanziellen Aufwand erfordert.

Knochenersatzmaterialien, die die an sie zu stellenden hohen biologischen und chemisch-physikalischen Voraussetzungen erfüllen, sind also eine medizinische und volkswirtschaftliche Notwendigkeit und das Entwicklungsziel zahlreicher Forschungsgruppen.

Im Laufe der Jahre wurden neben vielen anderen einige Implantatmaterialien entwickelt, die eine breite Anwendung gefunden haben und die den heutigen medizinischen und rechtlichen Ansprüchen an Medizinprodukte zwar weitgehend genügen, daneben aber die an derartige medizinische Präparate zu stellenden Anforderungen hinsichtlich Verfügbarkeit, Reinheit, Reproduzierbarkeit, Standardisierbarkeit, physikalisch-chemischer Stabilität oder Verträglichkeit nicht immer erfüllen. Insbesondere werden Hydroxylapatit-Keramiken, als im wesentlichen nicht abbaubare Produkte und in letzter Zeit in der Gruppe der resorbierbaren Knochenersatzstoffe wieder erneut in der Hauptsache Kalziumsulfat ("Plaster of Paris", Hemihydrat des Kalziumsulfats) häufiger verwendet.

Hydroxylapatit, das in kompakter und poröser Form, als stückiges Material oder als Granulat bzw. Pulver, zur Implantation kommt, ist praktisch nicht abbaubar und verbleibt daher nahezu unverändert auf immer im Organismus. Im Hinblick auf die knöcherne Integration sind es insbesondere poröse Implantate mit einem interkonnektierenden Hohlraumsystem, die, paßgenau in Form von Blöcken oder Zylindern implantiert, mit einem möglichst großflächigen innigen Verbund mit dem Wirtsknochen, die besten Ergebnisse erzielen. Entsprechende Tierversuche haben gezeigt, daß innerhalb einiger Monate die einen knöchernen Defekt ausfüllenden Implantate von den Kontaktflächen her von neugebildetem Knochen durchwachsen werden, der dabei die Oberfläche des Porensystems wie eine Tapete überzieht.

Der wesentliche Nachteil des Hydroxylapatits als Werkstoff ist seine materialimmanente Sprödigkeit, so daß derartige Implantate nie die mechanische Festigkeit - insbesondere nicht die Elastizität - des umgebenden Wirtsknochens erreichen, was ihren medizinischen Anwendungsbereich stark einengt. Zwar konnte gezeigt werden, daß nach ihrer Einheilung poröse Hydroxylapatitzylinder mechanisch fester waren, als die Ausgangsform, doch zeigte sich gleichzeitig, daß die Stege zwischen den Poren Risse und Spalten aufwiesen, die wiederum eine Schwächung des Implantats möglich erscheinen lassen. Da diese Art der Implantate dauerhaft im Organismus verbleiben, stellen sie auch auf Dauer Fehlstellen dar, die insbesondere in mechanisch stark belasteten Knochenbereichen zu einer dauerhaften Schwächung des Wirtsknochens und einem permanenten Frakturrisiko führen können.

Auf der anderen Seite verfügt Hydroxylapatit als Material über biologisch sehr positiv zu bewertende interaktive Eigenschaften mit dem Wirtsgewebe.

Um die Nachteile eines nicht resorbierbaren Knochenersatzes, die auch nach seiner völligen knöchernen Integration auf Dauer fortbestehen, zu umgehen, galten in den letzen Jahren die Forschungsbemühungen zunehmend abbaubaren Implantatmaterialien. Hierbei hat insbesondere Kalziumsulfat eine interessante Wiederentdeckung gefunden, liegt doch, neben vielen neueren Publikationen, ein erster Bericht bereits von 1892 vor, wo "Plaster of Paris" für die Auffüllung tuberkulöser bzw. osteomyelitischer Knochendefekte verwendet wurde.

Kalziumsulfat, das wie Hydroxylapatit über gute biologische Eigenschaften verfügt, wird allerdings - im Gegensatz zu diesem - im Organismus sehr rasch und in Abhängigkeit von Form und Volumen innerhalb weniger Wochen bis Monate vollständig abgebaut und resorbiert.

Diese an sich sehr erwünschte und vorteilhafte Eigenschaft wird allerdings dadurch konterkariert, daß das Implantat meist deutlich schneller abgebaut wird, als der Knochen vom Implantatbett aus nachwachsen kann, so daß in diesen Fällen wieder Hohlräume entstehen, die in der Regel dann nicht mehr von Knochen sondern von Bindegewebe ausgefüllt werden.

In der WO 87/05521 A1 wird ein Knochen-Implantatmaterial beschrieben, das als plastische, formbare Masse für die Auffüllung von Knochendefekten vorgesehen ist. Die Masse besteht aus gesintertem Hydroxylapatit-Granulat mit einer Korngröße von 250-5000µm, das weitgehend unlöslich bzw. im Organismus nicht abbaubar ist und Kalziumsulfat-Hemihydrat. Bei den beschriebenen Materialien handelt es sich um käufliche Produkte, die bezüglich ihrer Herstellung, den physikalisch-chemischen und medizinischen Eigenschaften und insbesondere ihrer Reinheit nicht näher definiert sind. Die trockenen Komponenten werden im Verhältnis von 70-60% zu 30-40% gemischt und mit einer geeigneten Flüssigkeit (Wasser, phisiolog. Kochsalzlösung) zu einer formbaren Masse angeteigt, die sich nach ihrer Applikation in situ verfestigt. Laut Beschreibung löst sich das Kalziumsulfat im Knochendefekt sehr rasch (innerhalb weniger Tage bis einiger Wochen) auf. Die auf diese Weise zwischen den dann mehr oder weniger lose im Defekt liegenden Hydroxylapatit-Granula entstehenden Hohlräume sollen den Einwuchs von neugebildetem Knochen ermöglichen. Aufgrund allgemein bekannter medizinischer Erkenntnisse aus den physiologischen Vorgängen bei der Knochenheilung erscheint das beschriebene Produkt, sowie seine Anwendungsweise, in verschiedener Hinsicht als problematisch. Bei der als Vorteil herausgestellten sehr raschen Auflösung des Kalziumsulfates besteht die Gefahr, dass dieses schneller gelöst ist, als neuer Knochen nachwachsen kann. In diesem Fall würden die Hydroxylapatit-Granulate, die keine gegenseitige Verbindung aufweisen und damit als Implantatmasse keinerlei mechanische Stabilität besitzen, durch Bindegewebe umwachsen und der Defekt würde nicht durch Knochen aufgefüllt. Diese Gefahr erscheint auch deshalb gegeben, weil sie Druckfestigkeit des Füllmaterials schon bei dieser in vivo-Prüfung in Kochsalzlösung bereits nach 2 Tagen um 53% reduziert war. Die Ergebnisse der beschriebenen Tierversuche sind nicht überzeugend. So werden "kleine Defekte (small holes)" in der Kaninchentibia erwähnt, die aber bekanntlich auch ohne Auffüllung im Zuge der physiologischen Reparaturvorgänge knöchern durchbaut werden. Auch die Defekte nach Zahnextraktionen bei Beagle-Hunden waren nur von geringer Größe. Mit und ohne Implantat waren die Defekte gleichermaßen nach 7-10 Tagen von Zahnfleisch überwachsen.

In den beiden Patentschriften EP 0 159 087 A1 und EP 0 159 089 A1 werden resorbierbare Implantatmaterialien als Wirkstoffträger für die Freisetzung von Medikamenten in Knochen oder Weichteilen beschrieben. Die Materialien bestehen jeweils aus zwei Komponenten. Im ersten Fall werden Kalziumsulfat- und Kalziumkarbonatpulver im Verhältnis 0,3 zu 1,0 und ein Medikament gemischt und dann mit Wasser angeteigt. Nach der Verfestigung der Masse kann sie z.B. als Antibiotikum-Träger in den Organismus verbracht werden. Durch den Zusatz von Kalziumkarbonat wird das Implantat sehr viel langsamer abgebaut als Gips (innerhalb mehrerer Monate), so dass für den Heilungsprozess genügend Zeit bleiben dürfte, um das Implantat z.B. in einem Knochendefekt durch neugebildeten Knochen zu ersetzen. Im zweiten Fall wird Kalziumsulfat mit Kalziummonohydrogenphosphat gemischt und die trockene Masse anschließend auf 700°C bis zur Schmelze des Kalziumhydrogenphosphates erhitzt. Die dabei entstehende mikroporöse Masse kann durch Imprägnation mit Medikamenten beschickt und als Medikamententräger in den Organismus verbracht werden. Keine der genannten Pulverkomponenten sind bezüglich ihrer chemisch-/physikalischen und/oder medizinischen Eigenschaften näher definiert.

Schließlich wird in der Patentanmeldung GB 2 323 083 A die Herstellung eines Implantatmaterials für die Auffüllung von Knochendefekten beschrieben, das aus einer Mischung von gesinterten oder nicht gesinterten Hydroxylapatit-Granula mit einer Größe von 100-400-5000 µm und einem Hydroxylapatitpulver mit einer Teilchengröße von 1-40 µm im Verhältnis von 1 : 0,1 bis 1 : 1 besteht. Diese Mischung wird trocken oder als wässrige Suspension gegebenenfalls unter Zusatz von Dispersions- oder Bindemitteln auf 800-1200°C erhitzt. Durch diesen Sintervorgang werden die größeren Granula durch das feine Pulver miteinander verbunden, so dass Formkörper (Blöcke) hergestellt werden können, die für die Auffüllung von Knochendefekten verwendet werden. Da unregelmäßige Knochendefekte nicht formschlüssig durch Blöcke aufgefüllt werden können, werden die Blöcke bei der Applikation durch Fingerdruck zerstört und so in die Ausgangsgranula zerlegt, die dann zur Defektauffüllung verwendet werden kann. Als gesintertes Hydroxylapatit ist das Implantatmaterial im Organismus nicht abbaubar. Ist schon aufgrund der bestehenden medizinischen Erkenntnisse die Auffüllung von Knochendefekten mit einzelnen größeren Granula wegen der damit verbundenen mechanischen Instabilität des Implantates problematisch und eine knöcherne Durchbauung des Implantates zu bezweifeln, so entbehrt das in der o.g. Patentschrift dargelegte Verfahren durchaus jeder Logik. Es muss nämlich gefragt werden, warum zunächst in einem aufwendigen Fertigungsprozess aus Granula Formkörper hergestellt werden, die als solche für die Defektauffüllung nur schlecht geeignet sind, um dann - zum Ausgleich dieses Nachteiles - bei der Applikation die Formkörper wieder zu zerstören, um die Ausgangsgranula darzustellen und zu implantieren.

Der Erfindung liegt die Aufgabe zugrunde, ein resorbierbares Implantatmaterial, insbesondere für die Verwendung in Chirurgie, Orthopädie, Traumatologie und im Dentalbereich, das die Vorzüge von Hydroxylapatit und Kalziumsulfat mit einander verbindet und gleichzeitig deren Nachteile vermeidet, sowie ein Verfahren zur Herstellung eines solchen Implantatmaterials zu schaffen.

Diese Aufgabe wird erfindungsgemäß dadurch gelöst, dass die pulverförmige Komponente im wesentlichen aus einer Mischung von Hydroxylapatit- und Kalziumsulfatpulfer besteht, wobei das Hydroxylapatitpulver aus synthetisch hergetellten gefällten hochreinen kristallinen Nano-Partikeln besteht, die eine Kristallgröße von 10-20 nm Breite und 50-60 nm Länge aufweisen.

Die spezifisch absorbierende BET-Oberfläche der Nano-Kristalle beträgt dabei 100-150 m²/g.

Das erfindungsgemäße Implantatmaterial muß dabei nicht unbedingt in Form von Formkörpern zum Zwecke der passgenauen Implantation vorliegen, weil deren "press fit" Implantation in der Praxis oft erhebliche Schwierigkeiten bereitet, sondern kann beispielsweise auch in Form einer sich nach der Applikation verfestigenden Masse implantierbar sein, was die Applikation sehr erleichtert und außerdem den Vorteil hat, daß auch sehr unregelmäßig begrenzte Defekte vollständig aufgefüllt werden können. Bei dieser Art der Defektauffüllung kann so auch das unerwünschte Einsprossen von Bindegewebe in die Defekte vermieden werden. Darüberhinaus soll das Material aber auch in Form eines Granulates bzw. kleiner bis größerer Formkörper, insbesondere der von Kugeln oder Zylindern einsetzbar sein, weil sich diese Anwendungsformen bei bestimmten Indikationen ebenfalls zur Defektauffüllung bewährt haben, sich darüber hinaus aber auch sehr gut als Wirkstoffträger eignen, wobei das Implantat selbst in jedem Fall aber in einer biologisch adäquaten Zeit abbaubar bzw. resorbierbar sein sollte.

Die beiden Komponenten der neuen, erfindungsgemäßen Knochenersatzmaterialien, Hydroxylapatit und Kalziumsulfat, sollen nachfolgend näher charakterisiert werden:

Es hat sich gezeigt, daß die erfindungsgemäße Forderung nach einem resorbierbaren Hydroxylapatit überraschend gut durch eine besondere Form eines Hydroxylapatits erfüllt werden kann. Bei dem erfindungsgemäß verwendeten Material handelt es sich um ein Hydroxylapatit, das im Gegensatz zu allen bisher als Implantate verwendete Hydroxylapatit-Materialien nicht einem Sinterungsprozess unterworfen wird, sondern auf synthetischem Weg durch einen Fällungsvorgang hergestellt wird. Das so gewonnene Material ist weiterhin dadurch charakterisiert, daß es aufgrund des besonderen Herstellungsverfahrens absolut rein ist und in Form kleinster Kristalle (sogenannter Nano-Partikel) vorliegt. Die Kristallgröße dieses Materials beträgt 10-20 nm Breite und 50-60 nm Länge, und kommt damit sehr nahe an die Größe der Apatitkristalle im menschlichen Knochen von 5-10 nm heran. Die spezifische absorbierende BET-Oberfläche der Nano-Kristalle beträgt ca.100-150 m²/g.

Ein derartiges kristallines Hydroxylapatitmaterial ist in der EP 0 664 133 B1 beschrieben. Allerdings wird in der genannten Patentschrift eine wässrige Paste mit einem Anteil von 18 - 36 Gew.% des genannten Hydroxylapatits beansprucht. Für eine derartige Paste konnten in standardisierten Tierversuchen sehr gute regenerative Eigenschaften in Bezug auf eine Stimulierung der körpereigenen Knochenheilung beobachtet werden. Voraussetzung war allerdings, daß die mit der Paste aufgefüllten, zylindrischen Defekte durch einen Deckel verschlossen wurden, so daß die Paste nach der Applikation nicht aus dem Defekt entweichen konnte.Der große Nachteil einer derartigen Anwendungsform in Gestalt einer wässrigen Paste liegt nämlich darin, daß sie, insbesondere bei größeren, sowie flachen, muldenartigen Defekten durch Blut- und/oder körpereigene Sekrete weiter verflüssigt und vorzeitig aus dem Defektlager ausgeschwemmt wird.

Demgegenüber wird beim erfindungsgemäßen Implantationsmaterial ein aus Hydroxylapatit-Nano-Partikeln bestehendes Pulver verwendet, das einerseits die guten biologischen Eigenschaften der Nano-Partikel besitzt, wobei es insbesondere wichtig ist, daß derartige Apatitkristalle, im Gegensatz zu gesinterten Hydroxylapatitkristallen, die im Körper nicht abgebaut werden, voll resorbierbar sind, auf der anderen Seite aber so lange die knöchernen Defekte ausfüllt und verschließt, bis in der Folge einer langsamen, physiologisch ablaufenden Resorption, das Implantat zeitgerecht durch neugebildeten, körpereigenen Knochen ersetzt wird.

Es wurde nun gefunden, daß zur Kombination mit den Hydroxylapatit-Nano-Partikeln zum Zwecke einer Verfestigung des Apatitpulvers, oder auch um aus diesem stückige Materialien, z.B. Granulate bzw. Formkörper für die Defektauffüllung bei besonderen medizinischen Indikationen, oder als Wirkstoffträger, ein auf synthetischem Weg hergestelltes Kalziumsulfat besonders geeignet ist. Ein solches erfindungsgemäßes Material besitzt außerdem den Vorteil, daß es - anders als die üblicherweise verwendeten "Plaster of Paris"-Materialien natürlicher Herkunft - als praktisch 100% reines, sehr hochwertiges Produkt, in gleichbleibender Qualität, reproduzierbar hergestellt werden kann und insbesondere keine im medizinischen oder physikalisch-chemischen Sinn unerwünschte oder schädliche Komponenten, Beimengungen oder Verunreinigungen enthält, so daß das Material standardisierbar ist und auf diese Weise insbesondere den Richtlinien der Medizinprodukteverordnung und des Arzneimittelrechts genügt.

Das beim erfindungsgemäßen Implantatmaterial verwendete Kalziumsulfatpulver besteht daher aus einem synthetisch hergestellten α-Subhydrat (Bassanit) mit nH₂O (wobei n<1 ist und sich 0,5 nähert) und in welchem zusätzlich ein Anteil von 5-10 % Kalziumoxid enthalten sein kann. Die spezifische, absorbierbare BET-Oberfläche beträgt 1,8-2,7, vorzugsweise 2,0-2,3 m²/g.

In entsprechenden Untersuchungen mit Materialien unterschiedlicher BET-Oberfläche hat sich überraschenderweise gezeigt, daß für den gewünschten biologischen bzw. therapeutischen Effekt der Kombination aus Apatit-Nano-Partikeln und dem beschriebenen Kalziumsulfat ein Verhältnis der spezifischen Oberflächen der zu kombinierenden Pulveranteile von Hydroxylapatit und Kalziumsulfat von ca. 150 : 2 ganz besonders vorteilhaft für die biologischen Effekte, sowohl der in situ sich verfestigenden Masse, als auch bei Anwendung in Form von stückigen Formkörpern, Granulaten, Kugeln, Zylindern etc. ist.

Weiterhin wurde gefunden, daß die sich nach der Implantation in den aufgefüllten Defekten verfestigende Masse vorteilhafterweise aus Hydroxylapatit-Nano-Partikel-Pulver mit einem Anteil von 15-45 %, vorzugsweise 20-30 %, des erfindungsgemäßen Kalziumsulfatpulvers besteht. Ein solches Pulvergemisch wird vor der Applikation mit 1-2 Teilen Wasser zu einer viskösen Masse verarbeitet, die sich nach der Implantation in situ verfestigt. Tierversuche haben gezeigt, daß nach der Applikation einer solchen Masse, diese als osteokonduktive Matrix das Einsprossen von Blutgefäßen und das Absiedeln knochenbildender Zellen ermöglicht, im knöchernen Defekt ein Zerfall der Matrix und deren Resorption einsetzt, wobei dieses Material die Knochenneubildung im Defektbereich stark anregt und insbesondere eine rasche Neoangiogenese, als Voraussetzung für jede anschließende knöcherne Konsolidierung, auslöst. Absolut überraschend und unerwartet und völlig abweichend von Erfahrungen mit anderen Hydroxylapatit-Implantaten hat sich dabei gezeigt, dass zu einem frühen Zeitpunkt bereits nach ca. 10 Tagen Vorläufer der knochenbildenden Zellen über die neugebildeten Kapillaren an den Implantationsort wandern, sich im Bereich der Apatit-Nanokristalle absiedeln und dort neuen Knochen bilden, der zunächst vorwiegend als Geflechtknochen vorliegt, sich dann aber in Lamellenknochen umwandelt. So konnte die übliche Zeit der Durchbauung eines knöchernen Defektes mit einem Durchmesser von 6 mm bei Kaninchen von 5-6 Wochen nach Auffüllung mit einer porösen gesinterten Keramik, nach Auffüllung mit einer Kombination aus Apatit-Nano-Partikeln und Kalziumsulfat auf 2,5-3 Wochen verkürzt werden. Der hohe Anteil von 70 - 80 Gew.% der Nano-Partikel in der Implantatmasse verhindert dabei einen zu raschen Abbau des Kalziumsulfats, so daß die Rate der Resorption des Implantates mit der physiologischen Rate des neugebildeten Knochens sehr gut zur Übereinstimmung gebracht wird.

Bei Verwendung des erfindungsgemäßen Kombinationsmaterial-Implantats ist dieser biologische Prozeß durch eine außerordentlich gute Verträglichkeit des Implantatmaterials gekennzeichnet, was sich insbesondere in dem Fehlen von Entzündungsreaktionen und durch das Ausbleiben von Serombildungen deutlich macht. Gerade derartige Reaktionen und insbesondere die häufig auftretenden Serome sind es, die bei anderen Implantatmaterialien den Heilungsverlauf - vor allem in den ersten 2 bis 3 Wochen - stören und damit auch die Knochenneubildung wesentlich verzögern. Bei herkömmlichen Kalziumsulfatimplantaten sind derartige Erscheinungen immer wieder beschrieben worden. Ein Phänomen, das mit dafür verantwortlich ist, daß Gips, trotz jahrzehntelanger klinischer Bemühungen, nie therapeutisch voll befriedigte und in den zurückliegenden Jahren keine breite Anwendung erfahren hat. Bei der Suche nach den Gründen hierfür hat sich gezeigt, daß offensichtlich der saure pH-Wert des Kalziumsulfats im Bereich von pH6 an diesen Vorgängen einen wesentlichen Anteil hat.

Überraschenderweise hat sich nun gezeigt, daß bei der Kombination der erfindungsgemäßen Materialien das Nano-Partikelpulver zu einer Verschiebung des pH-Wertes in den neutralen oder leicht alkalischen Bereich führt und dass von diesem Material eine Pufferwirkung ausgeht. Es ist seit langem bekannt, daß bei den biologischen Vorgängen der Gewebsregeneration und der Knochenheilung vom Organismus ein leicht alkalischer pH-Wert bevorzugt wird und für die Reparaturvorgänge sehr vorteilhaft ist. Insbesondere werden dadurch die Verzögerungen des Heilungsverlaufes, hervorgerufen durch Gewebeirritationen im sauren Milieu, vermieden, was das überraschend günstige, rasche und überlegene Heilungsmuster der erfindungsgemäßen Materialkombination erklärt. Dieser Effekt, nämlich das Erreichen eines leicht alkalischen pH-Wertes, wird durch einen geringen Anteil von 2-15 Gew.% an Kalziumoxid im Kalziumsulfat noch verstärkt.

Ein weiterer, ganz wesentlicher Vorteil im Vergleich zu bekannten Implantatmaterialien und insbesondere der Paste aus Nano-Partikeln ist, daß das erfindungsgemäße Knochenersatzmaterial infolge seiner verwendeten Kombinationspartner nach der Implantation im Röntgenbild sehr gut darstellbar ist.

In diesem Zusammenhang wurde weiterhin festgestellt, daß die erfindungsgemäße Masse unmittelbar nach dem Anmischen durch einen injektionsspritzen-ähnlichen Applikator mit entsprechend dimensionierter Kanüle, ggf. auch perkutan unter Röntgenkontrolle, in den knöchernen Defekt injiziert werden kann, wo sie dann zur Verfestigung gelangt.

Bei verschiedenen Indikationen im chirurgisch-orthopädischen, kieferchirurgischen und traumatologischen Bereich ist es vorteilhaft, anstelle oder zusätzlich zu einer plastischen Masse auch vorab verfestigtes stückiges Material, d.h. Formkörper wie z.B. Granulate, Kugeln, Zylinder, Prismen, Quader, etc., verschiedener Dimensionen für Defektauffüllungen und -sanierungen zur Verfügung zu haben.

Es hat sich nun gezeigt, daß - unter Aufrechterhaltung ihrer sehr vorteilhaften biologischen Eigenschaften - die erfindungsgemäße Kombination von Apatit-Nano-Partikeln und Kalziumsulfat für die Herstellung derartiger stückiger Formkörper verwendet werden kann.

In vielen Fällen kann es vorteilhaft sein, Implantatmaterialien, entweder zur Vermeidung von Infektionen im Gefolge der operativen Materialapplikation, oder zur sicheren Anwendung derartiger Implantate zur Sanierung osteomyelitischer Knochendefekte, mit einem oder mehreren geeigneten Antibiotika zu kombinieren. Auch wurde die Beladung derartiger Knochenersatzmaterialien mit anderen pharmazeutischen Wirkstoffen und Wachstumsfaktoren beschrieben.

Es hat sich nun gezeigt, daß die erfindungsgemäße Materialkombination für die Beschickung mit pharmazeutischen Wirkstoffen sehr gut geeignet ist. Durch die Mischung der beiden Komponenten mit Wasser können Formkörper verschiedener Größe und Gestalt hergestellt werden, die anschließend durch ein geeignetes Verfahren, wie beispielsweise einer Behandlung mit β-oder γ-Strahlen, oder die Begasung mit Ethylenoxid, sterilisiert werden können. Für die Sterilisation der Ausgangsmaterialien für die sich im Organismus verfestigenden Implantatmasse kommt eine Bestrahlung mit β-oder γ-Strahlen in Betracht.

Im Gegensatz zu bekannten Verfahren, bei denen beispielsweise dem Kalziumsulfatpulver oder dem Wasser Wirkstoffe zugesetzt werden, so daß diese bei der Herstellung des Materials in die Matrix gelangen (DE 196 20 117 C1; EP 0 159 087 A1) hat sich gezeigt, daß es sehr vorteilhaft ist, zunächst Granulate oder Formkörper aus den erfindungsgemäßen Materialkombinationen herzustellen, diese zu sterilisieren und dann erst mit geeigneten sterilen Wirkstofflösungen mit Antibiotika oder Wachstumsfaktoren als Wirkstoffen unter sterilen Bedingungen zu imprägnieren.

Überraschenderweise kann durch ein besonderes Mischungsverhältnis der beiden erfindungsgemäßen Komponenten und die Beobachtung des genannten bestimmten Verhältnisses der spezifischen BET-Oberflächen, sowie einem standardisierten Anmischverfahren, eine Matrix erzielt werden, die über eine standardisierbare und reproduzierbare Wasseraufnahmekapazität zwischen 46,6 und 68,3 Gew.% verfügt. Diese ermöglicht einerseits das problemlose und ebenso standardisierbare Tränken der Matrix mit den Wirkstofflösungen. Andererseits wurde gefunden, daß es auf diese Weise möglich ist, nicht nur durch die steuerbare Flüssigkeitsaufnahme die Beschikkungsmenge für die zu tränkenden Formkörper in engen Grenzen festzulegen, sondern daß auch die anschließende Wirkstofffreisetzung - in vitro, wie auch im biologischen Milieu nach der Implantation - reproduzierbar und insbesondere protrahiert erfolgt, wodurch mit derartigen Implantatkörpern gleichermaßen eine prophylaktische und/oder therapeutische Wirkung erzielt werden kann.

Außerdem besitzt dieses Implantatsystem den großen Vorteil, daß der behandelnde Arzt nicht nur - wie bei bekannten Materialien - ein Implantat mit nur einem vorgegebenen Wirkstoff in nur einer festgelegten Konzentration zur Verfügung hat, sondern anhand einer, auf dieses System abgestimmten Palette von sterilen Wirkstofflösungen in verschiedenen Konzentrationen, je nach Lage der individuellen klinischen Situation und ggf. des bakteriologischen Befundes eines bestimmten Patienten, unmittelbar vor der Implantation den günstigsten und für diese besondere Indikation geeignetsten Wirkstoff auswählen kann, die Tränkung unter sterilen Bedingungen mit Hilfe eines bereitgestellten Sets selbst vornimmt und dann das beschickte Material unmittelbar implantiert. Natürlich sind auf diese Weise auch Beladungen der Granulate oder Formkörper mit Wirkstoffkombinationen auf einfache Weise möglich, was dem Operateur eine sehr große therapeutische Breite und neue Möglichkeiten bei der gezielten lokalen Wirkstoffapplikation eröffnet.

## Patentansprüche

1. Aus einer Hydroxylapatit enthaltenden pulverförmigen Komponente und Flüssigkeit aufbereitetes resorbierbares Knochen-Implantatmaterial,
**dadurch gekennzeichnet,**
**dass** die pulverförmige Komponente im wesentlichen aus einer Mischung von Hydroxylapatit- und Kalziumsulfatpulver besteht, und
**dass** das Hydroxylapatitpulver aus synthetisch hergestellten, gefällten hochreinen kristallinen Nano-Partikeln besteht, die eine Kristallgröße von 10-20 nm Breite und 50-60 nm Länge aufweisen.

2. Implantatmaterial nach Anspruch 1, **dadurch gekennzeichnet, dass** die spezifische absorbierende BET-Oberfläche der Nano-Kristalle 100-150m²/g beträgt.

3. Implantatmaterial nach Anspruche 1 oder 2, **dadurch gekennzeichnet, dass** das Kalziumsulfatpulver aus einem synthetisch hergestellten, hochreinen Alfa-Subhydrat (Bassanit) mit nH₂O (wobei n<1 und annähernd 0,5 ist) besteht.

4. Implantatmaterial nach Anspruch 3, **dadurch gekennzeichnet, dass** die spezifische, absorbierende BET-Oberfläche der Kalziumsulfatpartikel 1,8-2,7 m²/g, vorzugsweise 2,0-2,3 m²/g beträgt.

5. Implantatmaterial nach Anspruch 3 oder 4, **dadurch gekennzeichnet, dass** das Kalziumsulfatpulver einen zusätzlichen Anteil von 2-15 Gew. %, vorzugsweise 5-10 Gew.%, Kalziumoxid enthält.

6. Implantatmaterial nach einem der Ansprüche 1 bis 5, **dadurch gekennzeichnet, dass** das BET-Oberflächenverhältnis der Mischung aus Apatit-Nano-Partikel und Kalziumsulfat-Partikeln etwa 150 : 2 beträgt.

7. Implantatmaterial nach einem der Ansprüche 1 bis 6, **dadurch gekennzeichnet, dass** der Anteil an Hydroxylapatitpulver in der pulverförmigen Komponente 85-55 Gew.%, vorzugsweise 80-70 Gew.%, beträgt.

8. Implantatmaterial nach einem der Ansprüche 1 bis 7, **dadurch gekennzeichnet, dass** die pulverförmige Komponente vor ihrer Aufbereitung durch β- oder γ-Strahlung sterilisiert ist.

9. Implantatmaterial nach Anspruch 8, **dadurch gekennzeichnet, dass** die sterile pulverförmige Komponente mit sterilem Wasser zu einer zunächst viskösen und sich dann verfestigenden Masse aufbereitet ist.

10. Implantatmaterial nach Anspruch 9, **dadurch gekennzeichnet, dass** der Anteil des sterilen Wassers bei der Aufbereitung der viskösen Masse 100-200 Gew.%, bezogen auf das Gewicht der pulverförmigen Komponenten beträgt.

11. Implantatmaterial nach Anspruch 9 oder 10, **dadurch gekennzeichnet, dass** die visköse Masse einen leicht alkalischen pH-Wert im Bereich von pH 7,5-8,2 aufweist.

12. Implantatmaterial nach einem der Ansprüche 9 bis 11, **dadurch gekennzeichnet, dass** die Masse nach der Aufbereitung eine fließfähige plastische Konsistenz aufweist.

13. Implantatmaterial nach einem der Ansprüche 1 bis 7, **dadurch gekennzeichnet, dass** es in Form von Granulaten verschiedener Korngrößen oder Formkörper, insbesondere Kugeln, Zylinder, Prismen oder Quader verschiedener Dimensionen vorliegt.

14. Implantatmaterial nach Anspruch 13, **dadurch gekennzeichnet, dass** die pulverförmigen Komponenten mit 100-200 Gew.% destilliertem Wasser, bezogen auf das Gewicht der pulverförmigen Komponenten, aufbereitet und in Formen verfestigt ist.

15. Implantatmaterial nach Anspruch 14, **dadurch gekennzeichnet, dass** das in den Formen verfestigte Material zu einer granulatförmigen Konsistenz zerkleinert ist.

16. Implantatmaterial nach einem der Ansprüche 13 bis 15, **dadurch gekennzeichnet, dass** es auf eine standardisierte reproduzierbare Wasseraufnahmekapazität eingestellt ist.

17. Implantatmaterial nach einem der Ansprüche 13 bis 16, **dadurch gekennzeichnet, dass** die Formkörper nach ihrer Aushärtung durch Bestrahlung mit β- oder γ-Strahlen oder durch die Begasung mit Ethylenoxid sterilisiert und steril abgepackt sind.

18. Implantatmaterial nach einem der Ansprüche 13 bis 17, **dadurch gekennzeichnet, dass** die Formkörper vor der Applikation mit sterilen pharmazeutischen Wirkstofflösungen getränkt sind.

19. Implantatmaterial nach Anspruch 18, **dadurch gekennzeichnet, dass** als Wirkstofflösungen flüssig aufbereitete Antibiotika oder Wachstumsfaktoren vorgesehen sind.

20. Implantatmaterial nach Anspruch 18 oder 19, **dadurch gekennzeichnet, dass** die Formkörper in Kombination aus zwei oder mehr verschiedenen Wirkstofflösungen getränkt sind.

21. Verfahren zur Herstellung eines Knochen-Implantatmaterials aus einer Hydroxylapatit enthaltenden pulverförmigen Komponente mit Wasser,
**dadurch gekennzeichnet,**
**dass** die pulverförmige Komponente durch Mischen von 85-55 Gew.% Hydroxylapatitpulver aus synthetisch hergestellten gefällten Nanopartikeln aus hochreinem Hydroxylapatit mit einer Kristallgröße von 10-20 nm Breite und 50-60 nm Länge mit 15-45 Gew.% Kalziumsulfatpulver in Form eines hochreinen α-Subhydrats mit nH₂O (wobei n<1 und annähernd 0,5 ist) hergestellt und die so entstandene Pulvermischung mit einem Anteil von 100-200 Gew.% sterilem Wasser zu einer zunächst viskösen Masse aufbereitet wird, die sich anschließend zu einem Körper verfestigt.

22. Verfahren nach Anspruch 21, **dadurch gekennzeichnet, dass** dem Kalziumsulfatpulver vor oder während des Vermischens mit dem Hydroxylapatitpulver 5-10 Gew.% pulverförmiges Kalziumoxid zugegeben wird.

23. Verfahren nach Anspruch 21 oder 22, **dadurch gekennzeichnet, dass** die pulverförmige Komponente vor ihrer Aufbereitung durch Bestrahlung mit β- oder γ-Strahlen sterilisiert wird.

24. Verfahren nach einem der Ansprüche 21 bis 23, dadurch kennzeichnet, dass die zunächst visköse aufbereitete Masse in Formen eingebracht, dort verfestigt und aus den Formen dann als stückige Formkörper ausgetragen wird.

25. Verfahren nach Anspruch 24, **dadurch gekennzeichnet, dass** die Formkörper durch Bestrahlung mit β- oder y-Strahlen oder durch eine Begasung mit Ethylenoxid sterilisiert und dann steril verpackt werden.

26. Verfahren nach Anspruch 24 oder 25, **dadurch gekennzeichnet, dass** die sterilen Formkörper vor der Applikation mit einer pharmazeutischen Wirkstofflösung getränkt werden.

27. Verfahren nach Anspruch 24 oder 25, **dadurch gekennzeichnet, dass** die sterilen Formkörper vor der Applikation mit einer Kombination aus zwei oder mehreren pharmazeutischen Wirkstofflösungen mit unterschiedlichen Wirkstoffen getränkt werden.

## Claims

1. Resorbable bone implant material prepared from a powdery component containing hydroxyl apatite and from a liquid,
**characterized in**
**that** the powdery component consists essentially of a mixture of hydroxyl apatite powder and calcium sulfate powder, and
**that** the hydroxyl apatite powder consists of synthetically produced, precipitated crystalline nanoparticles of high purity, which have a crystal size of 10-20 nm width and 50-60 nm length.

2. Implant material according to claim 1, **characterized in that** the specific absorbing BET surface area of the nanocrystals is 100-150 m²/g.

3. Implant material according to claims 1 or 2, **characterized in that** the calcium sulfate powder consists of a synthetically prepared alpha-subhydrate of high purity (bassanite) with n∗H₂O (wherein n<1 and approximately 0.5).

4. Implant material according to claim 3, **characterized in that** the specific absorbing BET surface area of the calcium sulfate particles is 1.8-2.7 m²/g, preferably 2.0-2.3 m²/g.

5. Implant material according to claim 3 or 4, **characterized in that** the calcium sulfate powder contains an additional fraction of 2-15 wt.%, preferably 5-10 wt.%, calcium oxide.

6. Implant material according to one of the claims 1 to 5, **characterized in that** the BET surface area ratio of the mixture of apatite nanoparticles and calcium sulfate particles is approximately 150 m²/g: 2m²/g.

7. Implant material according to one of the claims 1 to 6, **characterized in that** the fraction of hydroxyl apatite powder in the powdery components is 85-55 wt.%, preferably 80-70 wt.%.

8. Implant material according to one of the claims 1 to 7, **characterized in that** the powdery components is sterilized by β or γ radiation before its preparation.

9. Implant material according to claim 8, **characterized in that** the sterile powdery component is prepared with sterile water into an initially viscous and subsequntly solidifying mass.

10. Implant material according to claim 9, **characterized in that** the fraction of the sterile water during preparation of the viscous mass is 100-200 wt.%, based on the weight of the powdery components.

11. Implant material according to claim 9 or 10, **characterized in that** the viscous mass has a slightly alkaline pH value in the range of pH 7.5-8.2.

12. Implant material according to one of the claims 9 to 11, **characterized in that** the mass after preparaton has a viscous plastic consistency.

13. Implant material according to one of the claims 1 to 7, **characterized in that** the implant material is present in the form of granules having different grain sizes or in the form of form body, in particular sheres, cylinders, prisms or cuboids having different dimensions.

14. Implant material according to claim 13, **characterized in that** the powdery components are prepared with 100-200 wt.% distelled water, based on the weight of the powdery components, and solidified in molds.

15. Implant material according to claim 14, **characterized in that** the material that solidified in the molds is comminuted into a granular consistency.

16. Implant material according to one of the claims 13 to 15, **characterized in that** the implant material is adjusted to a standardized reproducible water retention capacity.

17. Implant material according to one of the claims 13 to 16, **characterized in that** the form body, after hardening, are sterilized by irradiation with β and γ rays or by gassing with ethylene oxide, and are packaged in sterile form.

18. Implant material according to one of the claims 13 to 17, **characterized in that** the form body are impregnated before application with sterile pharmaceutical solutions containing active ingredients.

19. Implant material according to claim 18, **characterized in that** antibiotics or growth factors prepared a liquid form are provided as solutions containing active ingredients.

20. Implant material according to claim 18 or 19, **characterized in that** the form body are impregnated with a combination of two or more different solutions containing acitve ingredients.

21. Method for preparing a bone implant material from a podery component containing hydroxyl apatite with water,
**characterized in**
**that** the powdery component is prepared by mixing of 85-55 wt.% hydroxyl apatite powder from synthetically produced, precipitated nanoparticles of high-purity hydroxyl apatite having a crystal size of 10-20 nm width and 50-60 nm lenght with 15-45 wt.% calcium sulfate powder in form of a highpurity α-subhydrate with nH₂O (wherein n<1 and approximately 0.5), and the produced powder mixture is prepared with a fraction of 100-200 wt.% sterile water into an initially viscous mass, which subsequently solidifies to form a body.

22. Method according to claim 21, **characterized in that** 5-10 wt.% cacium oxide powder is added to the calcium sulfate powder before or during mixing with the hydroxyl apatite powder.

23. Method according to claim 21 or 22, **characterized in that** the powdery components is sterilized before its preparation by irrediation with β or γ rays.

24. Method according to one of the claims 21 to 23, **characterized in that** the initially prepared viscous mass is introduced into molds, solidified therein and removed from the molds as pellet-like form body.

25. Method according to claim 24, **characterized in that** the form body are sterilized by irradiation with β or γ rays or by gassing with ethylene oxide and subsequently packaged in sterile form.

26. Method according to claim 24 oder 25, **characterized in that** the sterile form body are impregnated before the application with a pharmaceutical solution containing active ingredients.

27. Method according to claim 24 or 25, **characterized in that** the sterile form body are impregnated before the application with a combination consistings of two or more pharmaceutical solutions containing different active ingredients.

## Revendications

1. Matériau d'implant osseux résorbable préparé à partir d'un constituant en poudre contenant de l'hydroxylapatite et de liquide,
**caractérisé par le fait**
**que** le constituant en poudre est constitué essentiellement d'un mélange de poudre d'hydroxylapatite et de poudre de sulfate de calcium, et
**que** la poudre d'hydroxylapatite est constituée de nanoparticules cristallines de haute pureté précipitées produites par synthèse et ses cristaux ont de 10 à 20 nm de large et de 50 à 60 nm de long.

2. Matériau d'implant selon la revendication 1, **caractérisé par le fait que** l'aire massique BET absorbante des nanocristaux est de 100 à 150 m²/g.

3. Matériau d'implant selon l'une des revendications 1 et 2, **caractérisé par le fait que** la poudre de sulfate de calcium est constituée d'un sous-hydrate alpha (bassanite) de haute pureté produit par synthèse à n molécule d'eau (n étant inférieur à 1 et valant environ 0,5).

4. Matériau d'implant selon la revendication 3, **caractérisé par le fait que** l'aire massique BET absorbante des particules de sulfate de calcium est de 1,8 à 2,7 m²/g et de préférence de 2,0 à 2,3 m²/g.

5. Matériau d'implant selon l'une des revendications 3 et 4, **caractérisé par le fait que** la poudre de sulfate de calcium contient en plus de 2 à 15 % en poids, de préférence de 5 à 10 % en poids, d'oxyde de calcium.

6. Matériau d'implant selon l'une des revendications 1 à 5, **caractérisé par le fait que** le rapport des aires massiques BET du mélange de nanoparticules d'apatite et de particules de sulfate de calcium est d'environ 150/2.

7. Matériau d'implant selon l'une des revendications 1 à 6, **caractérisé par le fait que** la proportion de poudre d'hydroxylapatite dans le constituant en poudre est de 85 à 55 % en poids, de préférence de 80 à 70 % en poids.

8. Matériau d'implant selon l'une des revendications 1 à 7, **caractérisé par le fait que** le constituant en poudre est, avant sa préparation, stérilisé par rayonnement β ou γ.

9. Matériau d'implant selon la revendication 8, **caractérisé par le fait que** le constituant en poudre stérile est transformé avec de l'eau stérile en une matière d'abord visqueuse et ensuite se solidifiant.

10. Matériau d'implant selon la revendication 9, **caractérisé par le fait que** la proportion d'eau stérile pour la préparation de la matière visqueuse est de 100 à 200 % en poids par rapport au poids du constituant en poudre.

11. Matériau d'implant selon l'une des revendications 9 et 10, **caractérisé par le fait que** la matière visqueuse présente un pH légèrement alcalin compris entre 7,5 et 8,2.

12. Matériau d'implant selon l'une des revendications de 9 à 11, **caractérisé par le fait que** la matière présente après préparation une consistance plastique fluide.

13. Matériau d'implant selon l'une des revendications 1 à 7, **caractérisé par le fait qu'**il se présente sous forme de granulés de différentes grosseurs de grain ou de corps moulés, en particulier de sphères, cylindres, prismes ou parallélépipèdes rectangles de différentes dimensions.

14. Matériau d'implant selon la revendication 13, **caractérisé par le fait que** le constituant en poudre est préparé avec 100 à 200 % en poids d'eau distillée par rapport à son poids et solidifié dans des moules.

15. Matériau d'implant selon la revendication 14, **caractérisé par le fait que** la matière solidifiée dans les moules est broyée à une consistance de granulé.

16. Matériau d'implant selon l'une des revendications 13 à 15, **caractérisé par le fait qu'**il est ajusté à une capacité d'absorption d'eau reproductible normalisée.

17. Matériau d'implant selon l'une des revendications 13 à 16, **caractérisé par le fait que** les corps moulés sont, après leur durcissement, stérilisés par irradiation aux rayons β ou γ ou par gazage à l'oxyde d'éthylène et empaquetés de manière stérile.

18. Matériau d'implant selon l'une des revendications 13 à 17, **caractérisé par le fait que** les corps moulés sont, avant application, imprégnés de solutions d'agents pharmaceutiques stériles.

19. Matériau d'implant selon la revendication 18, **caractérisé par le fait que** comme solutions d'agents sont prévus des antibiotiques ou des facteurs de croissance préparés à l'état liquide.

20. Matériau d'implant selon l'une des revendications 18 et 19, **caractérisé par le fait que** les corps moulés sont imprégnés en combinaison de plusieurs solutions d'agents différentes.

21. Procédé de production d'un matériau d'implant osseux à partir d'un constituant en poudre contenant de l'hydroxylapatite avec de l'eau,
**caractérisé par le fait**
**que** le constituant en poudre est produit par mélange de 85 à 55 % en poids de poudre d'hydroxylapatite constituée de nanoparticules précipitées produites par synthèse d'hydroxylapatite de haute pureté à des cristaux de 10 à 20 nm de large et de 50 à 60 nm de long avec 15 à 45 % en poids de poudre de sulfate de calcium sous forme de sous-hydrate α de haute pureté à n molécule d'eau (n étant inférieur à 1 et d'environ 0,5), et le mélange de poudres ainsi obtenu est transformé avec 100 à 200 % en poids d'eau stérile en une matière d'abord visqueuse qui ensuite se solidifie en un corps.

22. Procédé selon la revendication 21, **caractérisé par le fait qu'**à la poudre de sulfate de calcium sont ajoutés, avant ou pendant le mélange avec la poudre d'hydroxylapatite, de 5 à 10 % en poids d'oxyde de calcium en poudre.

23. Procédé selon l'une des revendications 21 et 22, **caractérisé par le fait que** le constituant en poudre est, avant sa préparation, stérilisé par irradiation aux rayons β ou γ.

24. Procédé selon l'une des revendications 21 à 23, **caractérisé par le fait que** la matière préparée d'abord visqueuse est mise dans des moules, y est solidifiée et en est ensuite enlevée sous forme de corps moulés en morceaux.

25. Procédé selon la revendication 24, **caractérisé par le fait que** les corps moulés sont stérilisés par irradiation aux rayons β ou γ ou par gazage à l'oxyde d'éthylène et ensuite empaquetés de manière stérile.

26. Procédé selon l'une des revendications 24 et 25, **caractérisé par le fait que** les corps moulés stériles sont, avant application, imprégnés d'une solution d'agent pharmaceutique.

27. Procédé selon l'une des revendications 24 et 25, **caractérisé par le fait que** les corps moulés stériles sont, avant application, imprégnés d'une combinaison de plusieurs solutions d'agents pharmaceutiques contenant des agents différents.
